# EUROPEAN PATENT SPECIFICATION

(11) **EP 2 970 566 B1**
(45) Date of publication and mention of the grant of the patent: **21.02.2018**
(21) Application number: 14770348.2
(22) Date of filing: 14.03.2014
(51) Int. Cl.: C08G 59/32, C08G 59/62, C08G 18/09, C08G 18/70, C08L 5/04, C08G 59/42, A61L 31/10

(54) **MODIFIED HYALURONATE HYDROPHILIC COMPOSITONS, COATINGS AND METHODS**
MODIFIZIERTE HYDROPHILE HYALURONATZUSAMMENSETZUNGEN, BESCHICHTUNGEN UND VERFAHREN
COMPOSITIONS HYDROPHILES D'HYALURONATE MODIFIÉES, REVÊTEMENTS ET PROCÉDÉS

(30) Priority: 15.03.2013 US 201313834810
(43) Date of publication of application: 20.01.2016
(73) Proprietor: Lake Region Manufacturing, Inc. d/b/a Lake Region Medical, Chaska, MN 55318 (US)
(72) Inventor: EDWARDS, Peter, Anthony, Cokato, MN 55321 (US)
(74) Representative: Watson, Robert James
(86) International application number: PCT/US2014/027552
(87) International publication number: WO 2014/152632

(56) References cited:
- EP-B1- 1 023 090
- DE-U1-202004 009 060
- US-A1- 2010 189 758
- US-A1- 2010 216 951
- US-A1- 2012 041 554
- US-B2- 7 776 956

## Description

### BACKGROUND OF THE INVENTION

This invention relates to hydrophilic polymer compositions and a preferred application therefore, viz., hydrophilic medical device coatings.

Glycosaminoglycans, specifically including but not limited to hyaluronic acid ("HA") (also referred to as hyaluronan or hyaluronate) modified according to this invention provide a biocompatible, highly lubricious, durable, hydrophilic coating material. When used with, or crosslinked to, an optional base coat, the HA-based hydrophilic coatings of this invention can be applied to both metal and polymer substrates. Acutely-used i.e., intravascularly deployed for about 30 days or less, medical devices, e.g., catheters and guidewires, constitute a particularly preferred coating application for compositions of this invention.

### THE PRIOR ART

Hyaluronic acid, hyaluronate, hyaluronon, (including salts thereof) hereafter collectively "HA" is a naturally occurring high viscosity glycosaminoglycan having alternating β1-3 glucuronidic and β1-4 glucosaminidic bonds. The molecular weight of HA is generally within the range of <10,000 to 8,000,000 depending on the source, method of isolation and method of determination (Lifecore Biomedical web site). It is found in animal tissue, e.g., in umbilical cord, vitreous humor, synovial fluid, rooster combs, pathologic joints, group A and C hemolytic streptococci and in skin.

The isolation and characterization of HA is described in Meyer et al., J. Biol. Chem. 107, 629 (1934); J. Biol. Chem. 114, 689 (1936); Balazs, Fed. Proc. 17, 1086 (1958); Laurent et al.; Biochem. Biophys. Acta 42, 476 (1960). The structure of HA was elucidated by Weissman et al. J. Am. Chem. Soc. 76, 1753 (1954) and Meyer, Fed. Proc. 17, 1057 (1958). Cf., U.S. 4,141,973 Balazs. A representative structure of HA is shown in the Figures.

The application of unmodified HA to guidewires is noted in U.S. 6,042,876 to Deem

Belder and Malson et al. in U.S. 4,886,787 disclose Under alkaline conditions, ether linkages are generated from the di-epoxide hyaluronan reaction with hydroxyl functionality, while acid conditions form an ester.

Agerup in U.S. 4,886,787 discloses a process where HA is crosslinked with a di-epoxide.

The self-crosslinking reaction and oligomers of glycidyl carbamate ("GC") is described by Edwards et al. in Prog. Org. Coat., 57, 128-139 (2006).

Edwards et al. in JCT Research, 2(7), 517-528 (2005) describes reactions of glycidyl carbamate with various amines.

HYDAK A-16, is a coating composition available from Biocoat, Inc. is disclosed to comprise hyaluronic acid, polyacrylic acid and adjuvants, Cf., 6,673,453 to Beavers et al., col. 11, line 54. It is further noted in the '453 patent that free-acid form hyaluronic acid is obtainable from Biocoat, Inc. (col. 15, line 29).

DE 20 2004 009 060 U1 describes medical implantable devices coated with a carbon-containing layer which is produced by at least partially coating the device with a polymer film and heating the polymer film in an atmosphere, which is essentially free from oxygen, to temperatures in the region of 200 °C to 2500 °C.

Webster et al. in U.S. 7,776,956 (hereafter "the '956 patent") disclose water-dispersible epoxy urethane compounds and coating compositions.
The '956 patent discloses compositions comprising a polyfunctional oligomer having at least two epoxy urethane functional groups and a hydroxylated polyalkylene oxide chain. The compositions can be dispersed in water, with optionally added surfactants, to form a dispersion which is substantially free of volatile organic solvents.

No mention is made in the '956 patent of the possible use of the compositions of the patent to modify other polymers.

### BRIEF SUMMARY OF THE INVENTION

The present composition is particularly advantageously used as a top coat material on medical devices such as catheters, guidewires, or sheaths. The coating is advantageously hydrophilic, non-thrombogenic, lubricious and durable.

In a first aspect, the invention is an HA-based composition comprising the reaction product of a HA-compound selected from hyaluronic acid, a hyaluronate, a, hyaluronon, and salts thereof or an HA-compound selected from hyaluronic acid, a hyaluronate, a hyaluronon, and salts thereof coupled to polyalkylene glycol end-capped isocyanate; and a modified glycidol carbamate (GC) epoxide having its formula of I or II, or a self-crosslinked derivative thereof: 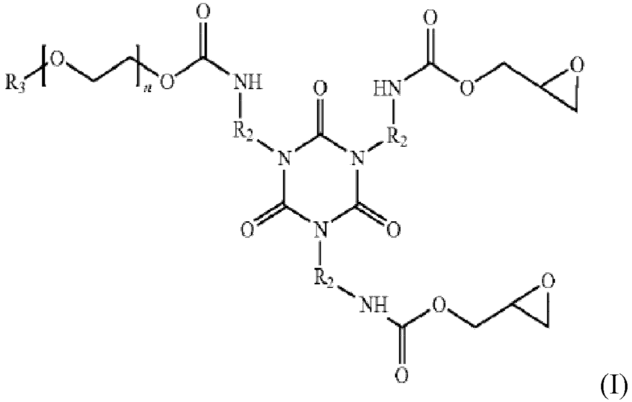 or 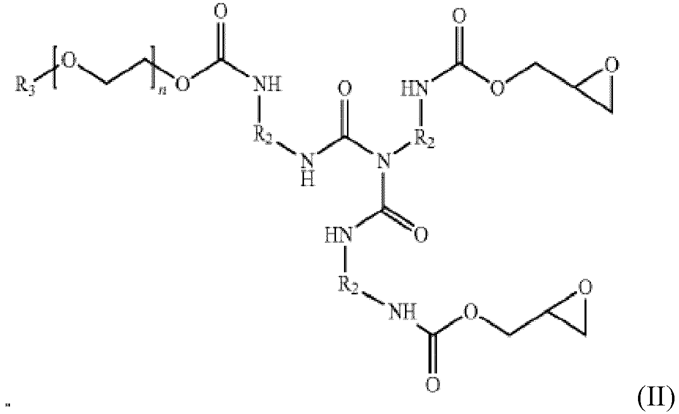
wherein n ranges from 1 to 50; R₂ is independently an optionally substituted divalent C₁-C₁₅ alkyl, an optionally substituted divalent C₃-C₁₅ cycloalkyl, or a group selected from: 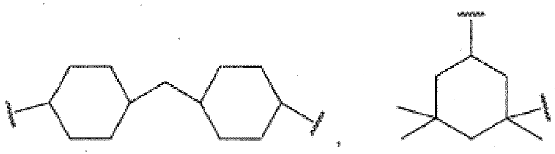 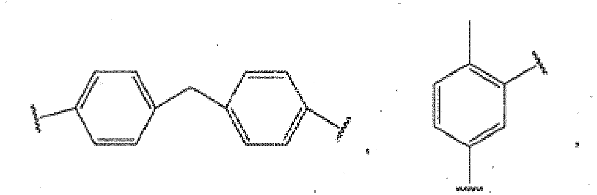 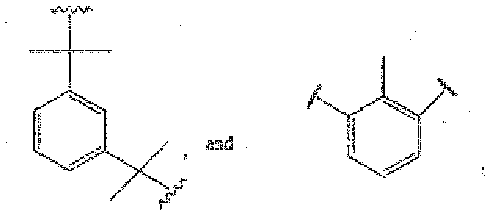
and R₃ is independently an optionally substituted C₁-C₁₅ alkyl.

In one embodiment of the invention, the modified glycidol carbamate epoxide is of formula I.

In another embodiment of the invention, the carbamate epoxide is of formula 1, n = 1, R₂ = (- CH₂-)₆ and R₃= - CH₂-.

In another embodiment of the invention, the GC moiety is self cross-linked.

In another embodiment of the invention, the glycidyl carbamate or polyurethane epoxy reacts with hydroxyl functionality of HA.

In another embodiment of the invention, the glycidyl carbamate or polyurethane epoxy reacts with carboxylic acid functionality of HA.

In a second aspect of the invention there is a method of coating a substrate with a water dispersible coating composition, comprising the step of applying the HA-based composition to a substrate.

In one embodiment of the invention, the substrate is selected from the group consisting of plastic or metal.

### BRIEF DESCRIPTION OF THE FIGS.

FIG. 1 shows the structural formula of "hyaluronic acid" (HA);
FIG. 2A and 2B show a general synthetic route for a composition of this invention;
FIG. 3 shows a likely structure of a modified HA material of this invention.

### DETAILED DESCRIPTION OF THE INVENTION

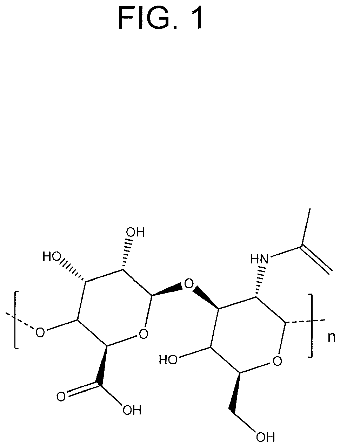

HA, for purposes of this invention, has a structural formula as shown in FIG. 1. As is noted above, the molecular weight of HA is in the range of <10,000 to as great as 8,000,000, or greater, meaning that "n" in this structural formula of FIG. 1 generally falls in the range of about <25 to about 25,000 or greater. It is to be understood that for purposes of this application the abbreviation "HA" is to be broadly construed to mean hyaluronic acid free and salts thereof, and hyaluronon or hyaluronate, and any of their commonly interchanged terms, which collectively refer to or references easily chemically interchanged species such as salts, esters, amides, acid halides and so forth. Generally speaking HA, as used herein, will have the structure shown in FIG. 1 and will have a structural formula of (C₁₄H₂₁NO₁₁)ₙ.

In an embodiment of this invention, HA, broadly construed, is partially or fully reacted with a composition of the '956 patent. The details of the chemistry of the '956 patent applicable to reaction with HA in accordance with this invention are set forth below.

In a further embodiment, a composition as described in the above paragraph is further reacted e.g., by heating, to induce self-crosslinking of a '956 patent moiety with a second '956 patent moiety.

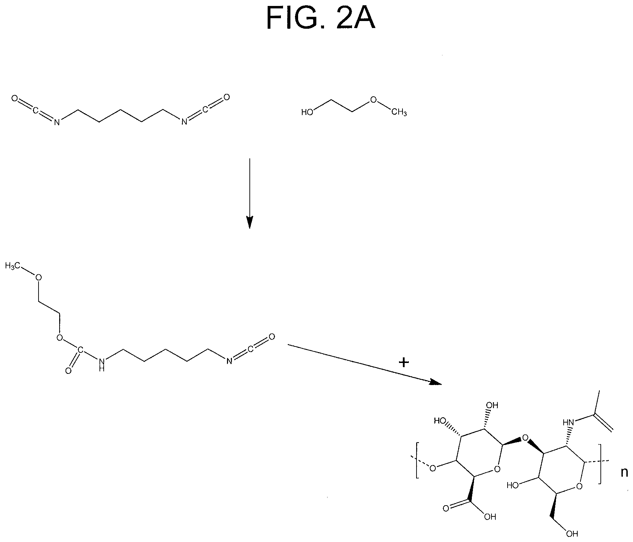

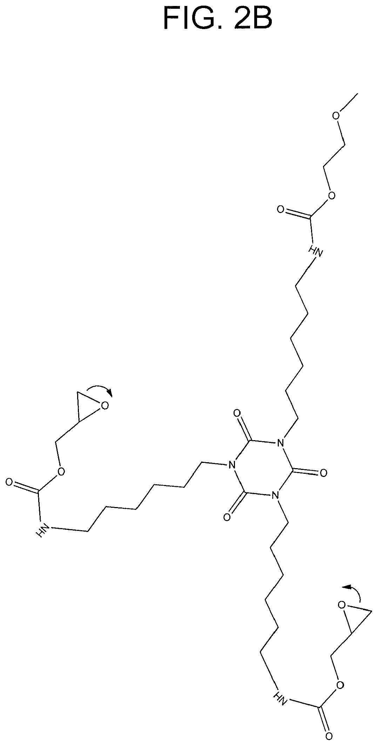

In yet a further embodiment HA, broadly construed, is reacted with an isocyanate, also broadly construed, which is mPEG end-capped, the reaction product being reacted with a composition of the '956 patent (FIGS. 2A, 2B) (synthetic details below).

According to this invention, HA, broadly construed, is modified by reacting it with an isocyanate prepolymer, or precursor which is a di-or poly-isocyanate, the di- or poly- isocyanate being end-capped with a segment, moiety or chain of methoxy poly (ethylene glycol), in abbreviated form referenced as "mPEG". mPEG has the structural formula of: 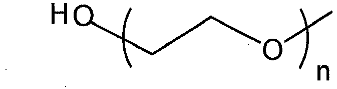 where "n" has a value of about 5 to 50, preferably about 12.5 to 42. It is to be understood that mPEG is a representative preferred example of the alkoxy polyalkylene glycol family of polymers.

Using a representative diisocyanate, e.g., hexamethylene diisocyanate, an isocyanate end-capped pre-polymer with mPEG has the formula: 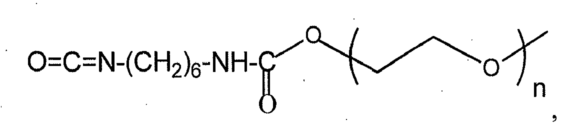 "n" having the values noted above.

Using a representative diisocyanate, e.g., hexamethylene diisocyanate, an isocyanate end-capped pre-polymer with glycidol has the formula:

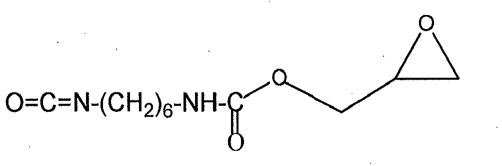

The above mPEG end-capped and glycidol end capped isocyanate mixed pre-polymer then is reacted with HA, noted above, to produce what is referenced here as "mPEG-NCO modified HA" according to this invention as follows:

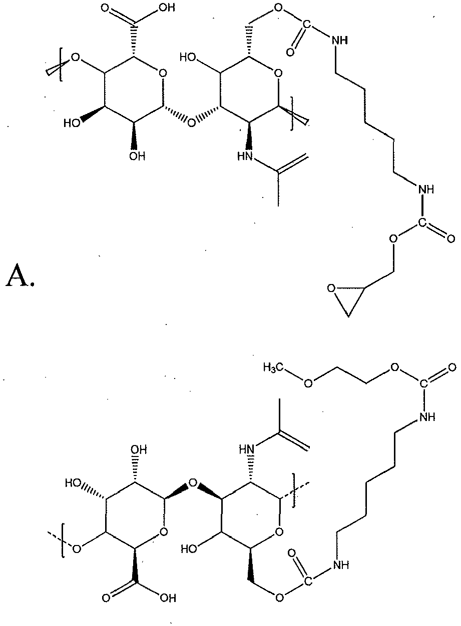

Mpeg and GC are mono-functional. GC is difficult to mix in alone, and thus requires a blend.

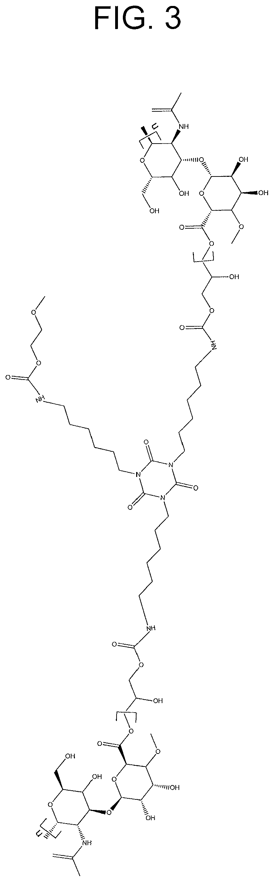

The oligomer of the'956 patent is fully reacted with HA, noted above, to produce what is referenced here as "'956 patent modified or partially modified HA" according to this invention. Partially modified HA can then be reacted further with HA or with externally added crosslinkers. The structural formula below (and FIG. 3) shows a likely structure of a modified HA material of this invention.

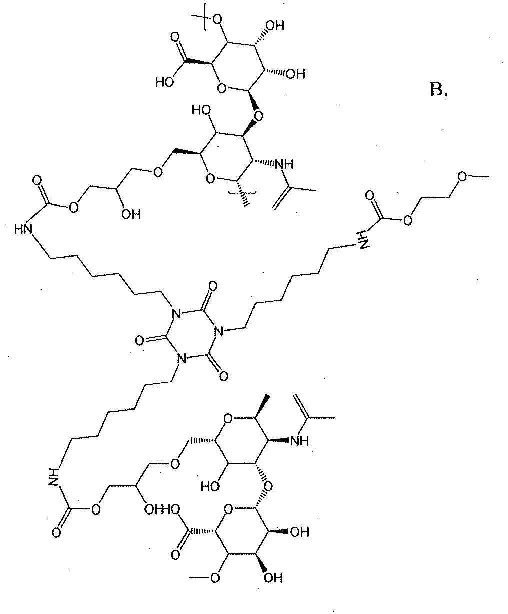

Without wishing to be bound by any theory it is theorized that the alcohol of Hyaluronan may help promote polyetherification or crosslink the Hyaluronan system.

The oligomer of the'956 patent is partially reacted with HA, noted above, to initiate the self-crosslinking reaction (temperatures >75C) to produce what is referenced here as "partially modified HA" according to this invention. The sequence below shows a likely structure of a modified HA material of this invention. During the modification of HA the polyurethane epoxy may undergo etherification or homopolymerization. This reaction may be initiated or involve Hyaluronan or self-crosslink of the GC moiety.

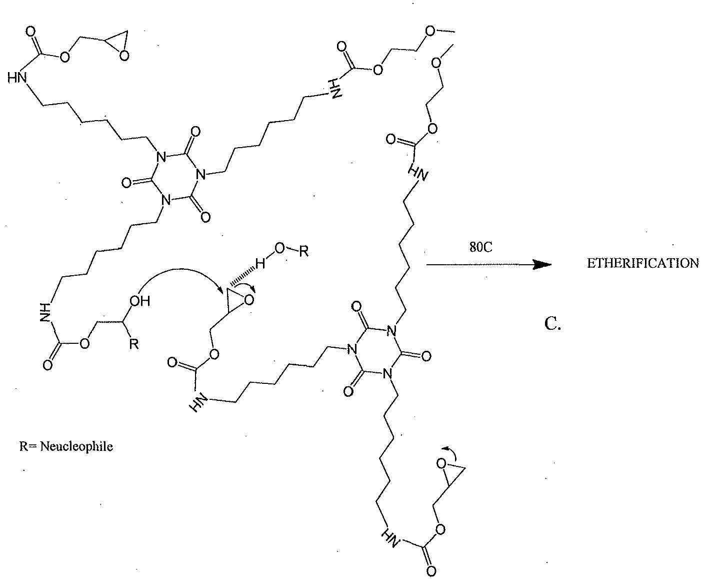

### OLIGOMERS OF THE '956 PATENT

The '956 patent discloses compositions comprising a polyfunctional oligomer having at least two epoxy urethane functional groups and a hydroxylated polyalkylene oxide chain. The compositions can be dispersed in water, with optionally added surfactants, to form a dispersion containing no volatile organic solvents.

The '956 patent discloses water-dispersible epoxy urethane resins of the Formula (I) or Formula (II). 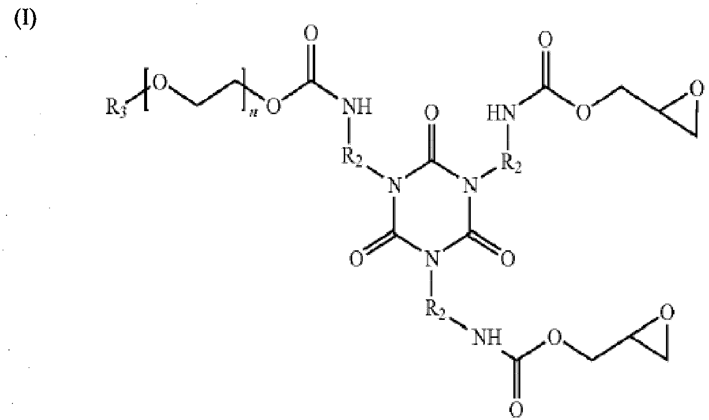 or 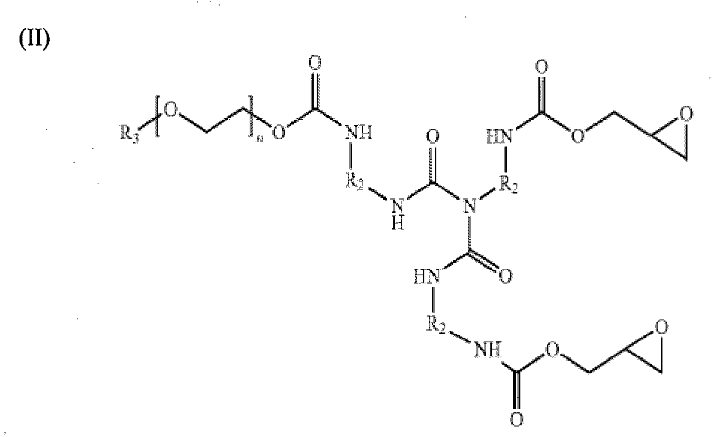 wherein
R₂ is independently an optionally substituted, divalent C₁-C₁₅ alkyl, optionally substituted divalent C₃-C₁₅ cycloalkyl, or a group selected from 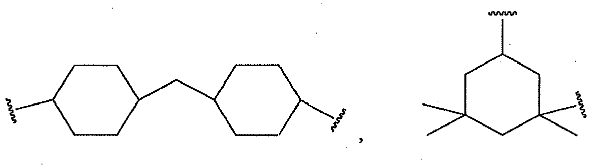 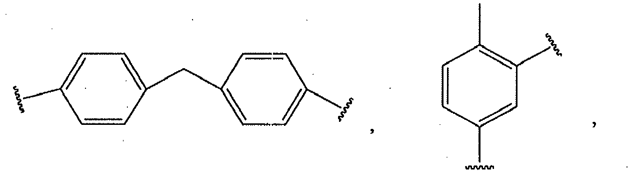 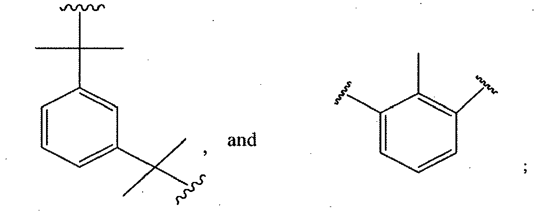
and R₃ is independently an optionally substituted C₁-C₁₅ alkyl or an optionally substituted divalent C₃-C₁₀ cycloalkyl.

In a preferred practice of the present invention, n = 1, R₂ = (- CH₂-)₆, and R₃ = - CH₃- is used.

Polyfunctional oligomers of the '956 patent are prepared from the reaction of hydrophilic modified polyfunctional resin having the difunctional isocyanates with glycidol. The polyfunctional resin is derived from controlled polymerization or oligomerization of the difunctional isocyanates. Free isocyanate is reacted with glycidol to form an epoxy urethane functional resin. The polyfunctional resin also includes a polyfunctional biuret.

Any suitable organic di-, tri-, or polyisocyanate, such as an aliphatic, cycloaliphatic, araliphatic or aromatic polyisocyanate, may be used either singly or in mixtures of two or more. The aliphatic isocyanates provide generally better light stability than the aromatic compounds. Aromatic polyisocyanates, on the other hand, are generally more economical and reactive toward polyols and other poly(active hydrogen) compounds than aliphatic polyisocyanates. Suitable aromatic polyisocyanates include but are not limited to those selected from the group consisting of 2,4-toluene diisocyanate, 2,6-toluene diisocyanate, a dimer of toluene diisocyanate (available under the Desmodur™ trademark from Bayer Materials Science, Leverkusen, Germany), diphenylmethane 4,4'-diisocyanate (MDI), 1,5-diisocyanato-naphthalene, 1,4-phenylene diisocyanate, 1,3-phenylene diisocyanate, fluorinated and/or silicone containing derivatives of the aforementioned, and mixtures thereof. Examples of useful cycloaliphatic polyisocyanates include but are not limited to those selected from the group consisting of dicyclohexylmethane diisocyanate (H₁₂ MDI, commercially available under the Desmodur™ trademark from Bayer Materials Science, Leverkusen, Germany), isophorone diisocyanate (IPDI), 1,4-cyclohexane diisocyanate (CHDI), 1,4-cyclohexanebis(methylene isocyanate) (BDI), 1,3-bis(isocyanatomethyl)cyclohexane (H₆ XDI), and mixtures thereof. Examples of useful aliphatic polyisocyanates include but are not limited to those selected from the group consisting of hexamethylene 1,6-diisocyanate (HDI), 1,12-dodecane diisocyanate, 2,2,4-trimethyl-hexamethylene diisocyanate (TMDI), 2,4,4-trimethyl-hexamethylene diisocyanate (TMDI), 2-methyl-1,5-pentamethylene diisocyanate, dimer diisocyanate, the urea of hexamethyl diisocyanate, and mixtures thereof. Examples of useful araliphatic polyisocyanates include but are not limited to those selected from the group consisting of m-tetramethyl xylylene diisocyanate (m-TMXDI), p-tetramethyl xylylene diisocyanate (p-TMXDI), 1,4-xylylene diisocyanate (XDI), 1,3-xylylene diisocyanate, or mixtures thereof.

Preferably, the polyfunctional resin derived from isocyanate or biuret is selected from the group consisting of TDI (toluene diisocyanate), TDI biuret, MDI (diphenylmethane diisocyanate), MDI biuret, HDI (hexamethylene diisocyanate), HDI biuret, NDI (naphthalene diisocyanate), NDI biuret, HMDI (hydrogenated MDI), HMDI biuret, and IPDI (isophorone diisocyanate) and IPDI biuret. More preferably, a polyfunctional resin derived from isocyanate or biuret consists of HDI (hexamethylene diisocyanate) or HDI biuret.

The polyfunctional oligomer of the invention is hydrophilic. Applicable hydrophilic functionality with suitable functional groups can readily be provided with the skilled person. Preferably, the polyfunctional oligomer has a polyalkylene oxide chain with 1 to 50 alkylene oxide units, preferably 2 to 20 alkylene oxide units. More preferably, the polyalkylene oxide chain may be an ethylene oxide chain, a propylene oxide chain, or an ethylene propylene oxide chain.

A preferred polyfunctional isocyanate resin based on hexamethylene diisocyanate and having ethylene oxide units is commercially available and sold under the Bayhydur XP 7165 tradename by Bayer Materials Science, Leverkusen, Germany.

The term "alkyl" includes straight and branched alkyl groups. The term "cycloalkyl", as used herein, refers to groups having three to ten, preferably three to seven carbon atoms. Suitable cycloalkyls include, but are not limited to cyclopropyl, cyclobutyl, cyclopentyl, cyclohexyl, cycloheptyl and the like. As indicated above, R₂ and R₃ may be substituted with any number of substituents or functional moieties. Examples of substituents include, but are not limited to, halo substituents, e.g. F; Cl; Br; or I; a hydroxyl group; a C₁-C₆ alkoxy group, e.g, --OCH₃, --OCH₂CH₃, or --OCH(CH₃)₂; a C₁-C₆ haloalkyl group, e.g.,--CF₃; --CH₂CF₃; or --CHCl₂; C₁-C₆ alkylthio; amino; mono and dialkyl amino groups; --NO₂; --CN; a sulfate group, and the like.

Optional surfactants are commonly used in coating formulations to improve wetting of the substrate by the coating, and wetting of the pigment by the resin. They can also improve formulating latitude by preventing shocking of the coating composition as various components are added and can increase the service life of the coating by increasing shelf stability. Typically, low levels of surfactants are used to accomplish these goals and mixtures of surfactants may be employed to impart one or more of the properties listed above. Surfactants are not generally volatile materials under ambient conditions and remain in the coating during the drying process. However, at the low concentrations typically used, little effect on polymer hardness or coating performance is observed. If too much surfactant is used in the aqueous coating composition, the wet coating could exhibit excessive foaming and poor thickening efficiency with thickeners while the cured coating could have problems with water sensitivity, poor exterior durability and poor block, stain and print resistance. Thus, surfactants are typically used in the lowest amounts necessary to achieve their beneficial properties while avoiding any detrimental effects.

Any anionic or nonionic surfactant, as well as mixtures, may be used in a water-based polymer coating composition of the invention. The surfactant is present in an amount effective to stabilize a coating formed from the composition. Preferably the nonionic surfactant is a polyether nonionic surfactant, more preferably, an alkyl polyglycol ether, an alkyl phenol polyglycol ether or a mixture thereof. Preferred alkyl phenol polyglycol ethers include ethoxylation products of octylphenol, nonylphenol, diisopropyl phenol, triisopropyl phenol or mixtures thereof. Preferred alkyl polyglycol ethers include ethoxylation products of lauryl alcohol, oleyl alcohol, stearyl alcohol or mixtures thereof. Preferred anionic surfactants include alkali metal or ammonium salts of alkyl, aryl or alkylaryl sulfonates, sulfates, phosphates. More preferably, the anionic surfactant is selected from sodium lauryl sulfate, sodium octylphenol glycolether sulfate, sodium dodecylbenzene sulfonate, sodium lauryldiglycol sulfate, ammonium tritertiarybutyl phenol and penta- and octa-glycol sulfonates, sulfosuccinate salts such as disodium ethoxylated nonylphenol half ester of sulfosuccinic acid, disodium n-octyldecyl sulfosuccinate, sodium dioctyl sulfosuccinate, and mixtures thereof. AEROSOL 18 surfactant, a 35% solution of disodium N-octyldecyl sulfosuccinimate in water and AEROSOL OT-75 surfactant, a 75% solution of sodium dioctyl sulfosuccinate in water, both available from Cytec Industries, Inc. are preferred anionic surfactants. Triton GR-7M is also preferred sulfosuccinate surfactant.

The HA-based coatings of the present invention can be coated upon bare metal substrates, i.e., metal surfaces of e.g., a medical devise. While not necessarily a preferred use of the compositions/coatings of this invention, application to bare metal are contemplated Generally, it is preferable to use some type of adhesion promoter on the metal itself. An adhesion promoter is generally much thinner than what is intended by the term "Base Coat" herein. Preferred adhesion promoters includes the triexthoxysilanes available from Gelest, Inc.

### BASE COAT (Optional)

Materials of this invention optionally may be applied over and reacted with (i.e., cross-linked to the) base coat materials of concurrently filed
application entitled "Oxirane Polyurethane Coatings," Attorney's Docket Number LRM-38016.

### EXAMPLE 1

### Procedure for Representative Synthesis of mPEG-NCO modified HA

1.1 Heat all reaction flasks and tops used for all reaction(s) at 100°C overnight to remove moisture.
1.2 Charge 1 mole, 162 grams hexamethylene dusocyanate (HDI), Sigma-Aldrich to a 50 mL cylindrical reaction vessel fitted into the appropriate heating mantel with a dry nitrogen purge.
1.4 Add 1/4 mole, 187.5 grams Methoxy (polyethylene glycol), Sigma-Aldrich, 750 Mn.
1.5 Let react for 10 minutes.
1.6 Next add 1/4 mole, 18.5 grams Glycidol carbamate, Dixie-Chemicals.
1.7 Mix this resin solution thoroughly at 500 RPM for 1 hr. and 30 min.
1.8 After 1 hour 30 min add 1 glass pipette drop of C - 95%.
1.9 After 2 hours increase the temperature of the J-KEM to 65C.
1.10 Hold reactor temperature, using the J-KEM and adjust with mixer RPM's to hold 80C for 1 hour for an NCO-mpeg and NCO-GC mixture.

### Modification Procedure of mPEG-NCO modified HA

2.1 Add NCO-mpeg and NCO-GC mixture (0.500±010 g) from previous step to a 4 ounce jar and record amount.
2.2 Next add HA, Shandong Topscience Biotech Co., Ltd (3.0±050 g) MW≥2 million Injection Grade, to the 4 ounce bottle and record amount.
2.3 Mix 4 ounce bottle contents at 500 RPM for 10 minutes to fully incorporate.
2.4 Move bottle side to side for full incorporation and to fully incorporate.
2.5 Place uncapped 4 ounce bottle into the oven set at 80C.
2.6 Heat at 80C for 2 hours for reaction to yield the mPEG-NCO modified HA component.

### EXAMPLE 2

### Procedure for Representative Synthesis of '956 modified HA

### 1.1 Synthesis as used in the '956 patent or materials purchased from Dixie-Chemicals as follows:

The following abbreviations and terms are used in the below:
HDI: hexamethylene diisocyanate
PACM: para amino-cyclohexyl methane
Anquamine 419: curing agent
MEK: Methyl ethyl ketone
ASTM: American Society for Testing and Materials
Materials

Glycidol was supplied by Dixie Chemical and stored refrigerated to minimize formation of impurities. An isocyanurate trimer of HDI (hexamethylene diisocyanate) with polyethylene oxide (Bayhydur XP 7165) was used as the polyfunctional isocyanate resin with an isocyanate equivalent weight of 230. K-KAT® XC-6212 was supplied by King Industries. Triton™GR-7M anionic surfactant was provided by Union Carbide and BYK 028 defoamer was provided by BYK Chemie USA. Amines used as hardeners were purchased from Aldrich and provided by Air Products. These include; bis(para-aminocyclohexyl) methane (PACM) and Anquamine 419, respectively. D.E.R® 332 (DGEBA) was supplied by The Dow Chemical Company.

### Synthesis of the Epoxy Urethane Resin

A 1000 ml four neck round bottom flask with condenser, nitrogen inlet and Model 210 J-KEM temperature controller, mechanical stirrer, with heating mantle were used for synthesis. The reaction vessel was charged with 225.21 grams glycidol and 700 grams of Bayhydur XP 7165 polyfunctional isocyanate resin and 0.112 grams K-KAT® XC-6212 (0.0025 weight percent). The temperature was held at 60°C. and the reaction was monitored and controlled within +/- two degrees Celsius. Infrared analysis was performed to determine reaction completion by monitoring the disappearance of the isocyanate peak at 2270 cm⁻¹. Epoxy equivalent weights were determined by titration with HBr (ASTM D1652). The theoretical epoxide equivalent weight of the product was 304, which compares with 303 grams/equivalent determined by titration.

Infrared (FTIR) measurements were performed using a Nicolet Magna-850 FTIR spectrometer. Sample aliquots were taken and coated directly on a potassium bromide salt plate. Omnic FTIR software for Nicolet was used to perform transmission with a final format of absorbance. Spectra acquisitions were based on 64 scans, resolution 4 and a data spacing of 1.98 cm.sup.-1. The main compartment was used and set for auto gain to monitor a spectral range of 4000 cm⁻¹ to 400 cm⁻¹. Different intervals of the reaction were sampled to monitor disappearance of the isocyanate peak. GRAMS 32 v5 FTIR software was employed for spectral calculations.

### Aqueous Coating Compositions

Aqueous coating compositions of the invention were formulated using the epoxy urethane resin of Example 1, an amine curing agent and water without addition of organic co-solvent. Coatings were formulated using 70% epoxy urethane resin and 30% water without addition of co-solvent. After mixing the resin and water, 1-drop Triton GR-7M surfactant was added for dispersion and diluting 1/6 of a drop of BYK 028 was used as a flow aid. Formulations were mixed with a glass stir rod, by hand, at room temperature. After the resin was dispersed in water, the amine curing agent, PACM or Anquamine 419, was added. Table 1 illustrates the formulation with actual amounts used. Coatings of from the aqueous coating compositions of the invention were prepared and tested as described below.

**TABLE 1**

| Formulation | Aqueous Coating Composition Formulation | | | | Solids (%) |
|---|---|---|---|---|---|
| | Water (g) | Resin (g) | PACM (g) | Anquamine 419 (g) | |
| 1 | 3.752 | 8.474 | 1.450 | - | 69.3 |
| 2 | 4.50 | 3.752 | - | 9.710 | 69.7 |

### Modification Procedure of '956 patent modified HA

2.1 Add '956 patent material above (0.002153 grams) to a 4 ounce jar and record amount.
2.2 Next add HA Supplier B (3.0±050 g) [MW≥2 million] [Injection Grade], to the 4 ounce bottle and record amount.
2.3 Mix 4 ounce bottle contents at 500 RPM for 10 minutes to fully incorporate.
2.4 Move bottle side to side for full incorporation and to fully incorporate.
2.5 Place uncapped 4 ounce bottle into the oven set at 80C.
2.6 Heat at 80°C for 2 hours for reaction to yield the '956 modified HA component.

### EXAMPLE 3

A guidewire coated with un-modified Hyaluronan is difficult to insert and withdrawal in a catheter. Modification, using Mpeg isocyanate and GC isocyanate, allows for a substantial drop in lubricity, which allows the catheter to guide easily. Without modification the guidewire cannot be inserted into the catheter without force. All coatings (Modified and Un-modified), shown in the paragraph below, contain '956 patent cross-linker where ARM was optional. In all cases, modification with Mpeg isocyanate and GC isocyanate, yields a very lubricious coating.

| Table of Modified Hyaluronan with Mpeg isocyanate and GC isocyanate and lubricity changes | | | | | | | | |
|---|---|---|---|---|---|---|---|---|
| | | Date | Lot | | MW | Vic | insertion | Withdrawal |
| HA Supplier A | ARM | Mar. 29, 2011 | 12424 | NO MODIFICATION | 2.59 M | 1448-1514 | 15.412 | 39.108 |
| HA Supplier A | NO ARM | Mar. 29, 2011 | 12424 | NO MODIFICATION | 2.59 M | 1448-1514 | 17.178 | 40.154 |
| HA Supplier A | ARM | Mar. 29, 2011 | 16066 | NO MODIFICATION | 741,000 | 78.2-83.6@ | 10.879 | 19.124 |
| HA Supplier A | NO ARM | Mar. 29, 2011 | 23588 | NO MODIFICATION | 1.60 M | 513-544 | 20.86 | 42.654 |
| HA Supplier A | ARM | Mar. 29, 2011 | 23588 | NO MODIFICATION | 1.50 M | 513-544 | 19.127 | 32.92 |
| HA Supplier B | ARM | Mar 29, 2011 | | NO MODIFICATION | 2.0 M | 831-861 | 21.298 | 36.243 |
| HA Supplier B | NO ARM | Mar. 29, 2011 | | NO MODIFICATION | 2.0 M | 831-861 | 20.653 | 37.592 |
| HA Supplier A | ARM | Mar. 30, 2011 | 16066 | MODIFIED | 741,000 | 82-90@12 F. | 13.723 | 18.567 |
| HA Supplier A | NO ARM | Mar. 30, 2011 | 16066 | MODIFIED | 741,000 | 82-90@12 F. | 13.82 | 17.261 |
| HA Supplier A | NO ARM | Mar. 30, 2011 | 23588 | MODIFIED | 1.60 M | 82-90@12 F. | 12 727 | 15.928 |
| HA Supplier A | ARM | Mar. 30, 2011 | 23588 | MODIFIED | 1.60 M | 82-90@12 F. | 10.848 | 13.793 |
| HA Supplier A | NO ARM | Mar. 30, 2011 | 12424 | MODIFIED | 2.59 M | 1494-1562 | 15.461 | 20.028 |
| HA Supplier A | ARM | Mar. 30, 2011 | 12424 | MODIFIED | 2.59 M | 1494-1562 | 15.86 | 18.292 |
| HA Supplier B | NO ARM | Mar. 30, 2011 | | MODIFIED | 2.0 M | 690-710 3.0 | 14.098 | 16.351 |
| HA Supplier B | ARM | Mar. 30, 2011 | | MODIFIED | 2.0 M | 690-710 3.0 | 12.014 | 15.96 |

The above insertion and withdrawal forces measurements (in grams) were taken using an Instron® tester using the same insertion and withdrawal procedure for each measurement. The guidewire on which the respective dip-coated compositions were placed had a tungsten loaded polyurethane jacket on which a base coat of concurrently filed application titled Oxirane (Ethylene Oxide) Polyurethane Coatings, Attorney's Docket No. LRM-38016 , had been deposited. Compositions of the present invention were then dip coated onto the base coat of the guidewire.

### EXAMPLE 4

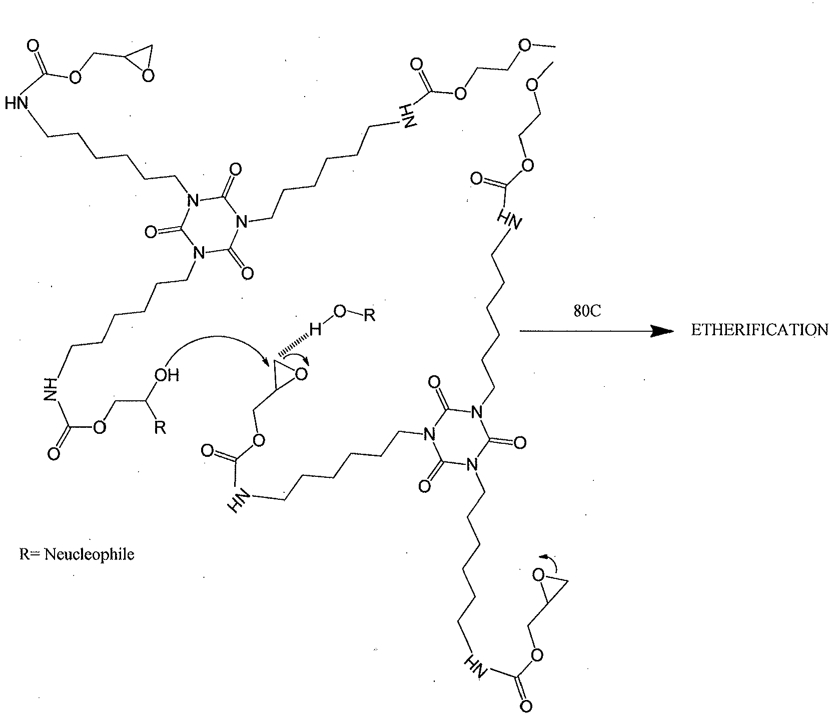

### Self-crosslinking reaction

During the modification of HA the polyurethane epoxy undergoes etherification or homopolymerization.

This reaction may be initiated or involve Hyaluronan or self-crosslink by itself.

### a. (Fully modified mpeg/GC-carbamate HA)

To a four ounce bottle 1.0 grams of mpeg/GC-carbamate were added to 3.0 grams Hyaluronan. The bottle was then placed into a 80C for 2.0 hours. The contents were then added to a 32 ounce bottle and filled with 700 grams distilled water. The material formed a hydrogel that was dissolved in water by mixing at 500 RPMs for 2-3 hours.

### b. (Partially modified HA)

To a four ounce bottle 0.005 grams of mpeg/GC-carbamate were added to 3.0 grams Hyaluronan. The bottle was then placed into an 80C oven for 2.0 hours. The contents were then added to a 32 ounce bottle and filled with 700 grams distilled water. The material formed a hydrogel that was dissolved in water by mixing at 500 RPMs for 2-3 hours.

### c. (Fully modified mpeg/GC-carbamate HA)

To a four ounce bottle 1.0 grams of mpeg/GC-carbamate were added to 3.0 grams Hyaluronan. The bottle was then placed into a 40C oven overnight. The contents were then added to a 32 ounce bottle and filled with 700 grams distilled water. The material formed a hydrogel that was dissolved in water by mixing at 500 RPMs for 2-3 hours.

### d. (Partially modified HA)

To a four ounce bottle 0.005 grams of mpeg/GC-carbamate were added to 3.0 grams Hyaluronan. The bottle was then placed into an 80C oven for two hours. The contents were then added to a 32 ounce bottle and filled with 700 grams distilled water. The material formed a hydrogel that was dissolved in water by mixing at 500 RPMs for 2-3 hours.

FTIR measurements were performed using a Shimadzu IRAffinity-1 with a MIRacle single-bounce ATR accessory using IRsolution software. Spectra acquisitions were based on 100 scans. The FTIR was set for auto gain to monitor spectral ranges of 4000 cm⁻¹ to 700 cm⁻¹.

Monitoring of the epoxide band (910 cm⁻¹) was used to determine reactions with hydroxyl functionality or the self-crosslinking reactions that may occur by neucleophilic attack. The disappearance of the epoxide absorbance band is a result of reaction completion. When presented and heated with hydroxyl functionality, from Hyaluronan, or alone the reaction proceeds to completion shows that molecular weight remains similar to unmodified Hyaluronan due to the system being slightly crosslinked.

### EXAMPLE 5

Higher amounts of '956 patent material modifier added to HA, then heated to 80C for 2 hrs, yield better lubricity and durability (Table 1.0). Notice withdrawal results for HA 30 are much lower than for HA 28 and HA 29. Withdrawal results correspond most to physician feel.

**Amt. '956**

| | Patent Material | Amt. HA | INS | WD | INS | WD |
|---|---|---|---|---|---|---|
| HA 30 coated at 0.15/4.5 | 2.5 g | 700 g | 9.877 | 10.486 | 12.067 | 11.785 |
| HA 29 coated at 0.15/4.5 | 1.0 g | 700 g | 10.968 | 12.165 | 15.537 | 14.019 |
| HA 28 coated at 0.15/4.5 | 0.0 g | 700 g | 10.399 | 10.594 | 16.211 | 15.86 |

### EXAMPLE 6

### Use of Adhesion Promoter on a Bare Metal Substrate

### Step 1: Preparation of Adhesion Promoter

1. 100 mL ETOH (ETOH anhydrous)
2. Add 5.0 gram water
3. Stir 10 minutes
4. Add 3.84 grams amino-silane
   1. Examples of amino-silane are:
      1. 3-aminopropyltriethoxysilane (Provided by Gelest)
      2. 3-aminoethyltriethoxysilane (Provided by Gelest)
      3. 3-trimethoxysilylpropyl-diethylenetriamine (Provided by Gelest)
5. Mix 5 minutes

### Step 2: Coating Wire Bare Metal Substrate with Adhesion Promoter

1. Pour adhesion promoter into 100 mL burette (use shaker to mix)
2. Place wire in graduated cylinder solution for 10 minutes
3. Take wire out of solution and place in 125 C oven for 10 minutes

## Claims

1. An HA-based composition comprising the reaction product of:
a)
(i) an HA-compound selected from hyaluronic acid, a hyaluronate, a, hyaluronon, and salts thereof; or
(ii) an HA-compound selected from hyaluronic acid, a hyaluronate, a hyaluronon, and salts thereof coupled to polyalkylene glycol end-capped isocyanate; and
b) modified glycidol carbamate (GC)epoxide having its formula of I or II, or a self-crosslinked derivative thereof: 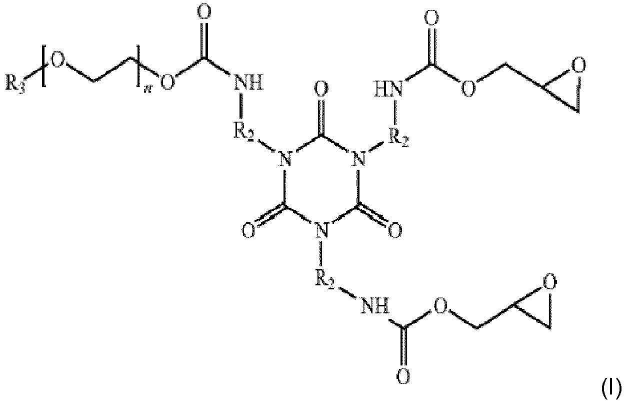 or 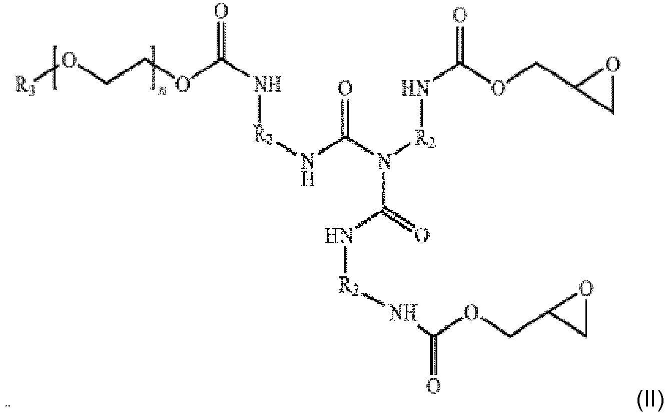 wherein:
(i) n ranges from 1 to 50;
(ii) R₂ is independently an optionally substituted divalent C₁-C₁₅ alkyl, an optionally substituted divalent C₃-C₁₅ cycloalkyl, or a group selected from: 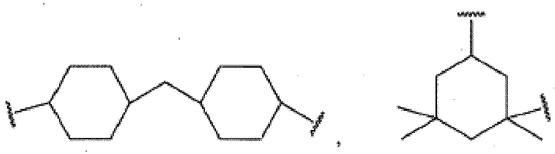 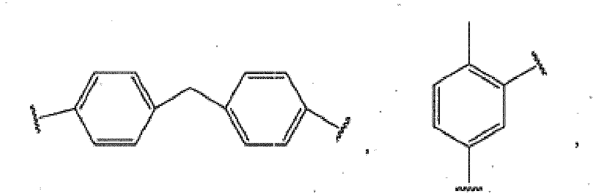 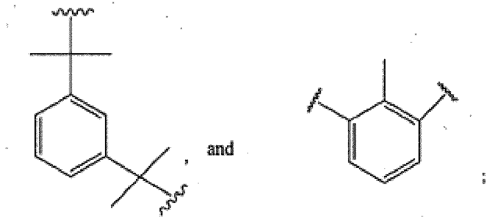 and
(iii) R₃ is independently an optionally substituted C₁-C₁₅ alkyl.

2. An HA-based composition according to claim 1 wherein the modified glycidol carbamate epoxide is of formula I.

3. An HA-based composition according to claim 2 wherein the carbamate epoxide is of formula 1, n = 1, R₂ = (- CH₂-)₆ and R₃= - CH₂-.

4. An HA-based composition according to claim 2 when the HA-compound is selected from hyaluronic acid, a hyaluronate, a, hyaluronon, and salts thereof, in which the GC moiety is self cross-linked.

5. An HA-based composition of claim 1 when the selected HA-compound is coupled to polyalkylene glycol end-capped isocyanate, wherein the glycidyl carbamate or polyurethane epoxy reacts with hydroxyl functionality of HA.

6. An HA-based composition of claim 1 when the selected HA-compound is coupled to polyalkylene glycol end-capped isocyanate, wherein the glycidyl carbamate or polyurethane epoxy reacts with carboxylic acid functionality of HA.

7. A method of coating a substrate with a water dispersible coating composition, comprising the step of applying the HA-based composition of claim 1 to a substrate.

8. The method of claim 7 wherein the substrate is selected from the group consisting of plastic or metal.

## Patentansprüche

1. HA-basierte Zusammensetzung, die das Reaktionsprodukt von Folgendem umfasst:
a)
(i) einer HA-Verbindung, ausgewählt aus Hyaluronsäure, einem Hyaluronat, einem Hyaluronon und Salzen davon; oder
(ii) einer HA-Verbindung, ausgewählt aus Hyaluronsäure, einem Hyaluronat, einem Hyaluronon und Salzen davon, die an mit Polyalkylenglykol endverkapptes Isocyanat gebunden sind; und
b) modifiziertes Glycidolcarbamat-(GC-)Epoxid, das die Formel I oder II aufweist, oder ein selbstvernetzendes Derivat davon: 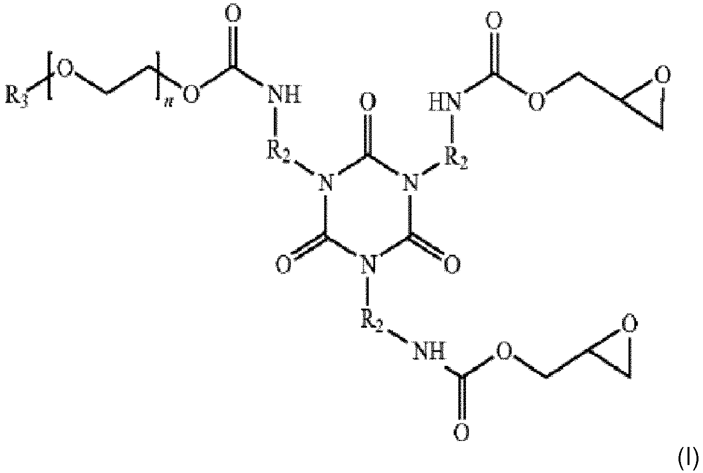 oder 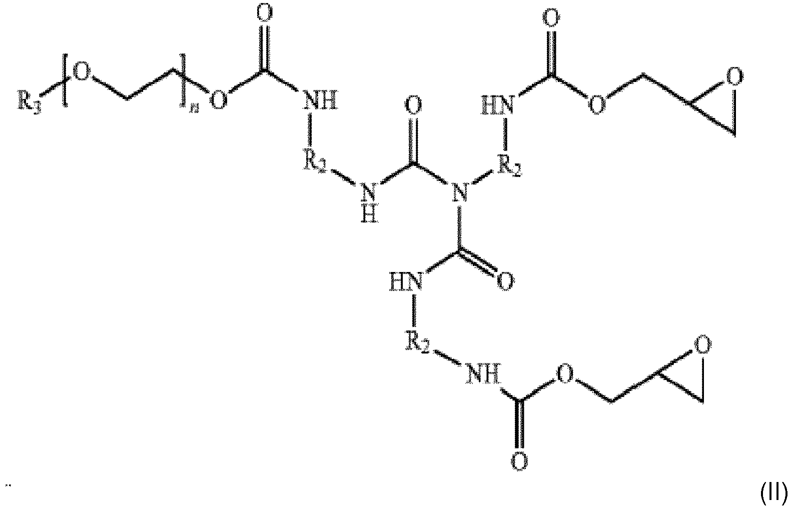 worin:
(i) n im Bereich von 1 bis 50 liegt;
(ii) R₂ unabhängig ein gegebenenfalls substituiertes zweiwertiges C₁-C₁₅-Alkyl, ein gegebenenfalls substituiertes zweiwertiges C₃-C₁₅-Cycloalkyl oder eine aus den folgenden ausgewählte Gruppe ist: 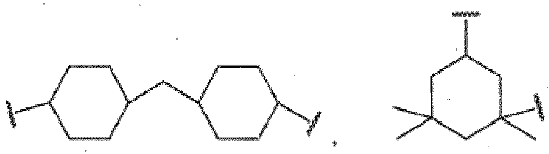 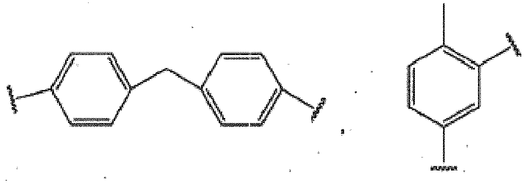 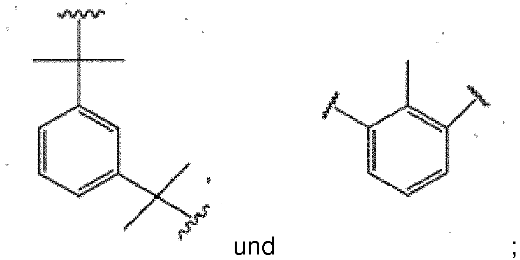 und
(iii) R₃ unabhängig ein gegebenenfalls substituiertes C₁-C₁₅-Alkyl ist.

2. HA-basierte Zusammensetzung nach Anspruch 1, wobei das modifizierte Glycidol-Carbamat-Epoxid die Formel I aufweist.

3. HA-basierte Zusammensetzung nach Anspruch 2, wobei das Carbamat-Epoxid die Formel 1 aufweist, worin n = 1 ist, R₂ (-CH₂-)₆ ist und R₃ -CH₂- ist.

4. HA-basierte Zusammensetzung nach Anspruch 2, wobei die HA-Verbindung aus Hyaluronsäure, einem Hyaluronat, einem Hyaluronon und Salzen davon ausgewählt ist, wobei die GC-Gruppierung selbstvernetzt ist.

5. HA-basierte Zusammensetzung nach Anspruch 1, wobei die ausgewählte HA-Verbindung an ein mit Polyalkylenglykol endverkapptes Isocyanat gebunden ist, wobei das Glycidyl-Carbamat oder Polyurethanepoxy mit der Hydroxylfunktionalität von HA reagiert.

6. HA-basierte Zusammensetzung nach Anspruch 1, wobei die ausgewählte HA-Verbindung an ein mit Polyalkylenglykol endverkapptes Isocyanat gebunden ist, wobei das Glycidyl-Carbamat oder Polyurethanepoxy mit der Carbonsäurefunktionalität von HA reagiert.

7. Verfahren zum Beschichten eines Substrats mit einer wasserdispergierbaren Beschichtungszusammensetzung, umfassend den Schritt des Auftragens einer HAbasierten Zusammensetzung nach Anspruch 1 auf ein Substrat.

8. Verfahren nach Anspruch 7, wobei das Substrat aus der aus Kunststoff und Metall bestehenden Gruppe ausgewählt ist.

## Revendications

1. Composition à base d'AH comprenant le produit réactionnel de :
a)
(i) un composé d'AH choisi parmi l'acide hyaluronique, un hyaluronate, un hyaluronane et les sels de ceux-ci ; ou
(ii) un composé d'AH choisi parmi l'acide hyaluronique, un hyaluronate, un hyaluronane et les sels de ceux-ci couplés à un isocyanate à extrémité coiffée par un polyalkylène glycol ; et
b) un époxyde de carbamate de glycidol (CG) modifié répondant à la formule I ou II, ou un dérivé auto-réticulé de celui-ci : 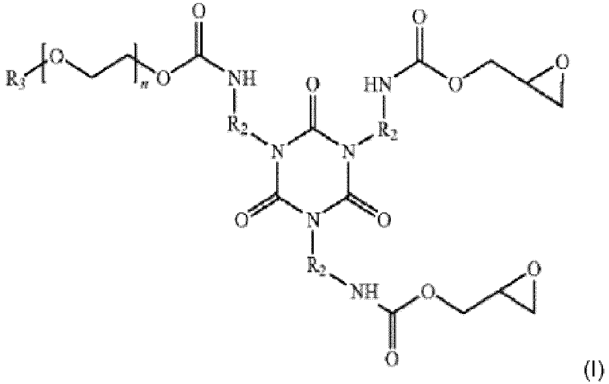 ou 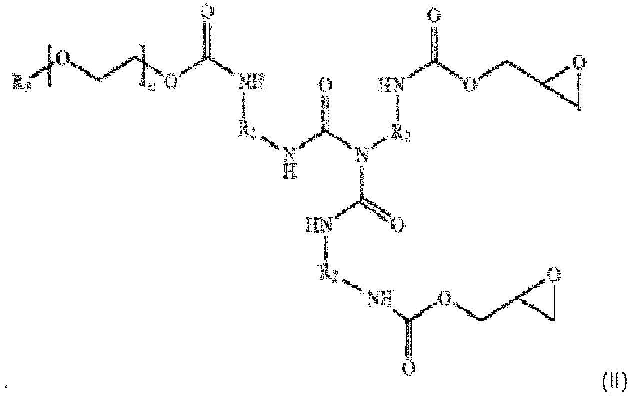 dans laquelle :
(i) n est situé dans la plage de 1 à 50 ;
(ii) R₂ est indépendamment un groupe alkyle divalent en C₁ à C₁₅ facultativement substitué, un groupe cycloalkyle divalent en C₃ à C₁₅ facultativement substitué, ou un groupe choisi parmi : 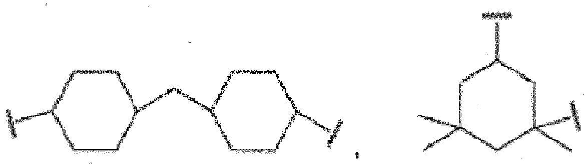 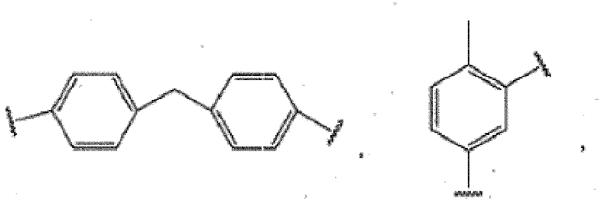 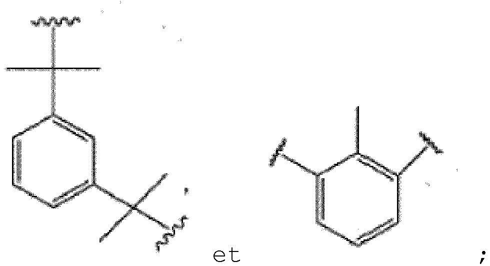 et
(iii) R₃ est indépendamment un groupe alkyle en C₁ à C₁₅ facultativement substitué.

2. Composition à base d'AH selon la revendication 1 dans laquelle l'époxyde de carbamate de glycidol modifié répond à la formule I.

3. Composition à base d'AH selon la revendication 2 dans laquelle l'époxyde de carbamate répond à la formule 1, n = 1, R₂ = (-CH₂-)₆ et R₃ = -CH₂-.

4. Composition à base d'AH selon la revendication 2 lorsque le composé d'AH est choisi parmi l'acide hyaluronique, un hyaluronate, un hyaluronane et les sels de ceux-ci, dans laquelle le groupement CG est auto-réticulé.

5. Composition à base d'AH selon la revendication 1 lorsque le composé d'AH choisi est couplé à un isocyanate à extrémité coiffée par un polyalkylène glycol, dans laquelle le carbamate de glycidyle ou le polyuréthane époxy réagit avec la fonctionnalité hydroxyle de l'AH.

6. Composition à base d'AH selon la revendication 1 lorsque le composé d'AH choisi est couplé à un isocyanate à extrémité coiffée par un polyalkylène glycol, dans laquelle le carbamate de glycidyle ou le polyuréthane époxy réagit avec la fonctionnalité acide carboxylique de l'AH.

7. Procédé de revêtement d'un substrat avec une composition de revêtement dispersible dans l'eau, comprenant l'étape d'application de la composition à base d'AH selon la revendication 1 sur un substrat.

8. Procédé selon la revendication 7 dans lequel le substrat est choisi dans le groupe constitué du plastique ou du métal.
